(19) Europäisches Patentamt / European Patent Office / Office européen des brevets

(11) **EP 2 123 441 B1**

(12) **EUROPEAN PATENT SPECIFICATION**

(45) Date of publication and mention of the grant of the patent:
**31.07.2013 Bulletin 2013/31**

(51) Int Cl.:
**B32B 5/26** (2006.01)    **D04H 3/00** (2012.01)
**D04H 3/14** (2012.01)    **D06M 17/00** (2006.01)
**D04H 1/559** (2012.01)    **D04H 1/4374** (2012.01)
**D04H 1/4358** (2012.01)

(21) Application number: **08712008.5**

(22) Date of filing: **26.02.2008**

(86) International application number:
**PCT/JP2008/053324**

(87) International publication number:
**WO 2008/108230 (12.09.2008 Gazette 2008/37)**

(54) **MIXED-FIBER NONWOVEN FABRIC LAMINATE**

MISCHFASER-VLIESSTOFFLAMINAT

STRATIFIÉ DE TISSU NON-TISSÉ À FIBRES MÉLANGÉES

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **02.03.2007   JP 2007053454**

(43) Date of publication of application:
**25.11.2009   Bulletin 2009/48**

(73) Proprietor: **Mitsui Chemicals, Inc.**
**Tokyo 105-7117 (JP)**

(72) Inventors:
• **MOTOMURA, Shigeyuki**
**Sodegaura-shi**
**Chiba 299-0265 (JP)**
• **SUZUKI, Kenichi**
**Sodegaura-shi**
**Chiba 299-0265 (JP)**
• **KUNIMOTO, Naosuke**
**Sodegaura-shi**
**Chiba 299-0265 (JP)**

(74) Representative: **HOFFMANN EITLE**
**Patent- und Rechtsanwälte**
**Arabellastrasse 4**
**81925 München (DE)**

(56) References cited:
JP-A- 2002 242 069    JP-A- 2003 096 653
JP-A- 2006 112 025    US-A1- 2004 067 710

• **"Fibers comprehensive dictionary", 2002, SEN'I SOUGOU JITEN HENSYU IINKAI ISBN: 4-88124-105-2 pages 418-419,**
• **"Non-woven fabric hand book", 1996, INDA * entry "Continuous filament" ***

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 123 441 B1

**Description**

Technical Field

The present invention relates to a mixed-fiber nonwoven fabric laminate that is excellent in strechability, flexibility, touch feeling, and formability and that is less sticky.

Background Art

Recently, since nonwoven fabrics are excellent in air permeability and flexibility, they are widely used in various applications. Accordingly, the nonwoven fabrics are required to have various characteristics according to the applications, and, at the same time, such characteristics are required to be improved.

For example, nonwoven fabrics used in sanitary materials, such as paper diapers and sanitary napkins, foundation cloths of poultices, and the like are required to have water resistance and also be excellent in moisture permeability. In addition, strechability and bulkiness are also required depending on the portions to be used.

As methods for imparting strechability to a nonwoven fabric, various methods are proposed. Examples of such methods include a method using a thermoplastic elastomer as a raw material of a spunbonded nonwoven fabric (for example, Patent Document 1: National Publication of International Patent No. 7-503502), a method using a fiber mixture of fibers comprising a thermoplastic polyurethane and fibers comprising a thermoplastic polymer as the fibers forming a nonwoven fabric (for example, Patent Document 2: Japanese Patent Laid-Open Publication No. 2004-244791), and, though the purpose is not the imparting of strechability, a continuous-fiber nonwoven fabric comprising a fiber mixture of adhesive fibers comprising, for example, a hydrogenated styrene block copolymer and non-adhesive fibers (for example, Patent Document 3: Japanese Patent Laid-Open Publication No. 2004-197291).

However, there is generally a tendency that a higher fiber mixing ratio of a thermoplastic elastomer improves the strechability, the flexibility, and the like, but deteriorates the formability, such as spinning stability, prevention of winding to a roller of forming apparatus, or winding to a nonwoven fabric-collecting belt.
[Patent Document 1] National Publication of International Patent No. 7-503502
[Patent Document 2] Japanese Patent Laid-Open Publication No. 2004-244791
[Patent Document 3] Japanese Patent Laid-Open Publication No. 2004-197291

Disclosure of Invention

Problems to be Solved by the Invention

It is an object of the present invention to develop a mixed-fiber nonwoven fabric laminate being excellent in strechability, flexibility, touch feeling, and formability and being less sticky.

Means for Solving the Problems

The present invention provides a mixed-fiber nonwoven fabric laminate including at least two mixed-fiber spunbonded nonwoven fabric layers whose fiber mixing ratios (in nonwoven fabric layer that is a mixture of two or more types of fibers, the content ratio of a specific type of fiber or the mixture ratio of each type of fiber in the nonwoven fabric layer is expressed as "fiber mixing ratio") of continuous fibers of a thermoplastic elastomer (A) and continuous fibers of a thermoplastic resin (B) other than (A) (hereinafter, also simply referred to as "thermoplastic resin (B)") are different from each other.

The present invention also provides a mixed-fiber nonwoven fabric laminate, wherein the at least two mixed-fiber spunbonded nonwoven fabric layers comprises a mixed-fiber spunbonded nonwoven fabric layer (C-1) and a mixed-fiber spunbonded nonwoven fabric layer (C-2). The mixed-fiber spunbonded nonwoven fabric layer (C-1) comprises the continuous fibers of thermoplastic elastomer (A) and the continuous fibers of thermoplastic resin (B) at a fiber mixing ratio of 40 to 95 wt% : 60 to 5 wt% (wherein, (A)+(B)=100 wt%). The mixed-fiber spunbonded nonwoven fabric layer (C-2) comprises the continuous fibers of thermoplastic elastomer (A) and the continuous fibers of thermoplastic resin (B) at a fiber mixing ratio of 10 to 60 wt% : 90 to 40 wt% (wherein, (A)+(B)=100 wt%).

Furthermore, the present invention provides a mixed-fiber nonwoven fabric laminate comprising at least three mixed-fiber spunbonded nonwoven fabric layers each comprising continuous fibers of a thermoplastic elastomer (A) and continuous fibers of a thermoplastic resin (B). In the laminate, the three mixed-fiber spunbonded nonwoven fabric layers are laminated such that the fiber mixing ratio of thermoplastic elastomer (A) in the mixed-fiber spunbonded nonwoven fabric layer (intermediate layer) located at the middle of the three mixed-fiber spunbonded nonwoven fabric layers is larger than those of the thermoplastic elastomer (A) in the other two layers.

Furthermore, the present invention provides a nonwoven fabric laminate comprising three mixed-fiber spunbonded nonwoven fabric layers, wherein the intermediate layer is preferably a mixed-fiber spunbonded nonwoven fabric layer (D-1) and the other two layers are preferably a mixed-fiber spunbonded nonwoven fabric layer (D-2) and a mixed-fiber spunbonded nonwoven fabric layer (D-3). The mixed-fiber spunbonded nonwoven fabric layer (D-1) comprises the continuous fibers of thermoplastic elastomer (A) and the continuous fibers of thermoplastic resin (B) at a fiber mixing ratio of 40 to 100 wt% : 60 to 0 wt% (wherein, (A)+(B)=100 wt%). The mixed-fiber spunbonded nonwoven fabric layer (D-2) comprises the continuous fibers of thermoplastic elastomer (A) and the continuous fibers of thermoplastic resin (B) at a fiber mixing ratio of 10 to 60 wt%: 90 to 40 wt% (wherein, (A)+(B)=100 wt%). The mixed-fiber spunbonded nonwoven fabric layer (D-3) comprises the continuous fibers of thermoplastic elastomer (A) and the continuous fibers of thermoplastic resin (B) at a fiber mixing ratio of 10 to 60 wt%: 90 to 40 wt% (wherein, (A)+(B)=100 wt%).

Effects of the invention

The mixed-fiber nonwoven fabric laminate of the present invention has both characteristics of having excellent strechability, flexibility, touch feeling, and formability and of being less sticky.

Best Modes for Carrying Out the Invention

Thermoplastic elastomer (A)

As a thermoplastic elastomer (A) that serves as a continuous-fiber raw material of the thermoplastic elastomer (A) that is one of fiber components forming a mixed-fiber spunbonded nonwoven fabric layer according to the present invention, various known thermoplastic elastomers can be used and may also be used in a combination of two or more.
Examples of the thermoplastic elastomer (A) include block copolymers comprising a polymer block of at least one aromatic vinyl compound such as styrene and a polymer block of at least one conjugated diene compound such as a butadiene or isoprene, represented by a polystyrene-polybutadiene-polystyrene block copolymer (referred to as SBS), a polystyrene-polyisoprene-polystyrene block copolymer (referred to as SIS), hydrogen additives thereof, namely, polystyrene-polyethylene/butylene-polystyrene block copolymer (referred to as SEBS) and polystyrene-polyethylene/propylene-polystyrene block copolymer (referred to as SEPS); styrene elastomers represented by hydrogen additives thereof; polyester elastomers represented by block copolymers comprising highly crystalline aromatic polyester and noncrystalline aliphatic polyether; polyamide elastomers represented by block copolymers comprising crystalline polyamide having a high melting point and noncrystalline polyether or polyester having a low glass transition temperature (Tg); thermoplastic polyurethane elastomers represented by block copolymers comprising a hard segment of polyurethane and a soft segment of, for example, polycarbonate polyol, ether polyol, caprolactone polyester, or adipate polyester; polyolefin elastomers, for example, noncrystalline or low-crystalline random copolymers that are obtained by singly using ethylene/$\alpha$-olefin random copolymers, propylene/$\alpha$-olefin random copolymers, propylene/ethylene/$\alpha$-olefin random copolymers, or the like, mixtures of the noncrystalline or low-crystalline random copolymer and a propylene homopolymer, and mixtures of the noncrystalline or low-crystalline random copolymer, a copolymer of propylene and a small amount of $\alpha$-olefin, and crystalline polyolefin such as high-density polyethylene or middle-density polyethylene; polyvinyl chloride elastomers; and fluorine elastomers.
Examples of the styrene elastomers include polystyrene block- and butadiene rubber block- or isoprene rubber block-based diblock and triblock copolymers. The rubber blocks may be unsaturated or completely hydrogenated. Specifically, the styrene elastomers are manufactured and marketed under trade names such as KRATON polymer (trade name, manufactured by Shell Chemical Company), SEPTON (trade name, manufactured by Kuraray Co. Ltd.), TUFTEC (trade name, manufactured by Asahi Kasei Corporation), and Leostomer (trade name, manufactured by Riken Technos Corporation).
The polyester elastomers are specifically manufactured and marketed under trade names such as HYTREL (trade name, manufactured by E.I. DuPont & Company) and Pelprene (trade name, manufactured by Toyobo Co., Ltd.).
The polyamide elastomers are specifically manufactured and marketed under trade names such as Pebax (trade name, manufactured by Atofina Japan Co., Ltd.).
Examples of the polyolefin elastomers include ethylene/$\alpha$-olefin copolymers and propylene/$\alpha$-olefin copolymers. Specifically, the polyolefin elastomers are manufactured and marketed under trade names such as Tafmer (trade name, manufactured by Mitsui Chemicals Inc.), Engage (trade name, manufactured by DuPont Dow Elastomers L.L.C.), which is an ethylene-octene copolymer, CATALLOY (trade name, manufactured by Montell), which contains a crystalline olefin copolymer, and Vistamaxx (trade name, manufactured by ExxonMobil Chemical Corporation).
The polyvinyl chloride elastomers are specifically manufactured and marketed under trade names such as Leonyl (trade name, manufactured by Riken Technos Corporation) and Posmere (trade name, manufactured by Shin-Etsu Polymer Co., Ltd.).

Among these thermoplastic elastomers, the thermoplastic polyurethane elastomers are preferred in terms of strechability and processability.

Thermoplastic polyurethane elastomer

Among the thermoplastic polyurethane elastomers, those having a solidification starting point of 65°C or higher, preferably 75°C or higher, and most preferably 85°C or higher are preferred. The upper limit of the solidification starting point is preferably 195°C. The solidification starting point used herein is determined with a differential scanning calorimeter (DSC) and is the starting temperature of an exothermic peak, which is attributed to the solidification of the thermoplastic polyurethane elastomer and is observed when the thermoplastic polyurethane elastomer is heated to 230°C at a rate of 10°C/min, held at 230°C for 5 minutes, and then cooled at a rate of 10°C/min. A thermoplastic elastomer having a solidification starting point of 65°C or higher can suppress defects such as fusion bonded fibers, broken fibers, and resin masses when a mixed-fiber spunbonded nonwoven fabric is produced and also can prevent the resulting mixed-fiber spunbonded nonwoven fabric from winding to an embossing roller during hot embossing. In addition, the resulting mixed-fiber spunbonded nonwoven fabric is less sticky and is therefore suitably used for materials that will be in direct contact with the skin, such as garments, sanitary materials, and sports materials. Furthermore, the forming processability can be enhanced by controlling the solidification starting point to 195°C or lower. The solidification starting point of the resulting fibers tends to be higher than that of the thermoplastic polyurethane elastomer used.

In order to adjust the solidification starting point of such a thermoplastic polyurethane elastomer to 65°C or higher, it is necessary to select a polyol, an isocyanate compound, and a chain extender, which are used as raw materials of the thermoplastic polyurethane elastomer, so as to each have an optimum chemical structure and also necessary to control the amount of hard segments. The amount of hard segments herein is a weight percent (wt%) value calculated by dividing the total weight of the isocyanate compound and the chain extender used for producing the thermoplastic polyurethane elastomer by the total weight of the polyol, the isocyanate compound, and the chain extender, and multiplying the quotient by 100. The amount of the hard segments is preferably 20 to 60 wt%, more preferably 22 to 50 wt%, and most preferably 25 to 48 wt%.

The number of polar solvent-insoluble particles of the thermoplastic polyurethane elastomer is preferably 3000000 or less per gram, more preferably 2500000 or less, and still more preferably 2000000 or less. The polar solvent insolubles of the thermoplastic polyurethane elastomer herein are mainly agglomerates such as fish eyes and gels that are generated during the production of the thermoplastic polyurethane elastomer. The agglomerates are components generated from the raw materials of the thermoplastic polyurethane elastomer and reactions of these materials, for example, a component derived from aggregated hard segments of the thermoplastic polyurethane elastomer and a component of hard segments and/or soft segments crosslinked with, for example, an allophanate bond or a biuret bond.

The number of the polar solvent-insoluble particles is the number of insolubles generated when the thermoplastic polyurethane elastomer is dissolved in a dimethylacetamide (hereinafter, abbreviated as "DMAC") solvent and is counted with a particle size distribution analyzer, which utilizes a pore electric resistance method, equipped with an aperture of 100 $\mu$m in diameter. The use of 100 $\mu$m aperture allows counting of particles of 2 to 60 $\mu$m in size in terms of uncrosslinked polystyrene.

Problems, such as an increase in fiber diameter distribution and broken fibers during spinning, can be further suppressed by controlling the number of the polar solvent-insoluble particles to 3000000 or less per one gram of the thermoplastic polyurethane elastomer in the above-mentioned range of the solidification starting point of the thermoplastic polyurethane elastomer. From the viewpoints of suppressing the incorporation of bubbles to the strands or the occurrence of broken fibers during the production of a nonwoven fabric with a large spunbonding machine, the water content in the thermoplastic polyurethane elastomer is preferably 350 ppm or less, more preferably 300 ppm or less, and most preferably 150 ppm or less.

In addition, from the viewpoint of strechability, the thermoplastic polyurethane elastomer preferably satisfies the following formula (I):

$$a/(a+b) \leq 0.8 \qquad (I),$$

more preferably the following formula (II):

$$a/(a+b) \leq 0.7 \qquad (II),$$

and

most preferably the following formula (III):

$$a/(a+b) \leq 0.55 \qquad (III),$$

wherein "a" is the total of heat of fusion determined from endothermic peaks within the peak temperature range of 90°C to 140°C and "b" is the total of heat of fusion determined from endothermic peaks within the peak temperature range of higher than 140°C to 220°C when the thermoplastic polyurethane elastomer is observed by thermal analysis with a differential scanning calorimeter (DSC).

In the above formulae, "a/(a+b)" means a ratio (unit: %) of heat of fusion of a hard domain of the thermoplastic polyurethane elastomer. When the ratio of heat of fusion of the hard domain of the thermoplastic polyurethane elastomer is 80% or less, the fibers, in particular, the fibers of the mixed-fiber spunbonded nonwoven fabric, and the nonwoven fabric are improved in strength and strechability. In the present invention, the lower limit of the ratio of heat of fusion of the hard domain of the thermoplastic polyurethane elastomer is preferably about 0.1%.

The melt viscosity of such a thermoplastic polyurethane elastomer is preferably 100 to 3000 Pa·s, more preferably 200 to 2000 Pa·s, and most preferably 1000 to 1500 Pa·s when measured under conditions of a temperature of 200°C and a shear rate of 100 sec$^{-1}$, from the viewpoint of suppressing the occurrence of broken fibers. The melt viscosity herein is a value measured with a Capirograph (manufactured by Toyo Seiki K.K., nozzle length: 30 mm, diameter: 1 mm).

The thermoplastic polyurethane elastomer having these characteristics can be produced by, for example, a method as described in Japanese Patent Laid-Open Publication No. 2004-244791.

The mixed-fiber spunbonded nonwoven fabric produced using the above-described thermoplastic polyurethane elastomer is excellent in touch feeling and, therefore, can be suitably used as, for example, a sanitary material.

In the thermoplastic polyurethane elastomer whose amount of polar solvent-insoluble substances is small, the filter installed in an extruder for filtering impurities and so on is hardly clogged during the production of mixed-fiber spunbonded nonwoven fabrics, resulting in decreases in frequencies of adjustment and maintenance of the apparatus. Therefore, as described below, the thermoplastic polyurethane elastomer that is obtained by filtration after polymerization of a polyol, an isocyanate compound, and a chain extender is also industrially preferred.

Polyolefin elastomer

Among the polyolefin elastomers, preferred are those being noncrystalline or low-crystalline and preferably having an X-ray diffraction crystallinity of 20% or less (including 0%), and examples thereof include ethylene/α-olefin copolymers of ethylene and one or more α-olefins having 3 to 20 carbon atoms (such as propylene, 1-butene, 1-pentene, 1-hexene, 4-methyl-1-pentene, 1-heptene, 1-octene, and 1-decene); and propylene/α-olefin copolymers of propylene and one or more α-olefins having 2 to 20 (excluding 3) carbon atoms (such as ethylene, 1-butene, 1-pentene, 1-hexene, 4-methyl-1-pentene, 1-heptene, 1-octene, and 1-decene). Furthermore, the polyolefin elastomer may contain, in addition to the α-olefins above, a small amount of a cyclic olefin such as norbornene, 5-methylnorbornene, 5-ethylnorbornene, cyclopentene, 3-methylcyclopentene, or 4-methylcyclopentene; a cyclic diene such as 5-ethylidene-2-norbornene, 5-mthylene-2-norbornene, 1,3-cyclopentadiene, 1,3-cyclohexadiene, or 1,4-cyclohexadiene; or a vinyl compound such as vinyl acetate or methacrylic acid ester. (Herein, when the polyolefin is a copolymer, the constituent unit including the monomer mentioned first is the main component of the copolymer.)

Specific examples of the noncrystalline or low-crystalline ethylene/α-olefin copolymers include ethylene/propylene random copolymers and ethylene/1-butene random copolymers. Furthermore, the melt flow rate (MFR) of the ethylene/α-olefin copolymer is not particularly limited as long as the copolymer is spinnable, but the MFR (ASTM D1238, 190°C, a load of 2160 g) is usually in the range of 1 to 1000 g/10 min, preferably 5 to 500 g/10 min, and more preferably 10 to 100 g/10 min.

Specific examples of the noncrystalline or low-crystalline propylene/α-olefin copolymers include propylene/ethylene random copolymers, propylene/ethylene/1-butene random copolymers, and propylene/1-butene random copolymers. Furthermore, the MFR of the propylene/α-olefin copolymer is not particularly limited as long as the copolymer is spinnable, but the MFR (ASTM D1238, 230°C, a load of 2160 g) is usually in the range of 1 to 1000 g/10 min, preferably 5 to 500 g/10 min, and more preferably 10 to 100 g/10 min.

The polyolefin elastomer may be the single noncrystalline or low-crystalline polymer as described above, and may also be a composition containing about 1 to 40 wt% of crystalline polyolefin such as a propylene homopolymer, a copolymer of propylene and a small amount of α-olefin, high-density polyethylene or medium-density polyethylene in addition to the noncrystalline or low-crystalline polymer.

A particularly preferred polyolefin elastomer composition is an elastomer composition comprising a polypropylene resin composition that contains 1 to 40 wt% of an isotactic polypropylene (i) and 60 to 99 parts by weight of a propylene/ethylene/α-olefin copolymer (ii) (copolymer of 45 to 89 mol% of propylene, 10 to 25 mol% of ethylene, and α-olefin having 4 to 20 carbon atoms) (wherein, the copolymerization amount of the α-olefin having 4 to 20 carbon atoms is at most 30 mol%).

Thermoplastic resin (B)

The thermoplastic resin (B) is a raw material for continuous fiber comprising a thermoplastic resin that is one of the components forming a mixed-fiber spunbonded nonwoven fabric constituting the nonwoven fabric laminate of the present invention. Various known thermoplastic resins other than the thermoplastic elastomers (A) can be used as the thermoplastic resin (B), and examples thereof include crystalline polymers having a melting point (Tm) of 100°C or higher and noncrystalline polymers having a glass transition temperature of 100°C or higher. Among these thermoplastic resins (B), the crystalline thermoplastic resins are preferred.

In addition, the thermoplastic resin (B) is preferably a thermoplastic resin (elongation thermoplastic resin) having characteristics showing substantially no elastic recovery and being capable of imparting a maximum point elongation of 50% or more, preferably 70% or more, and more preferably 100% or more to a nonwoven fabric produced therefrom by a known spunbonded nonwoven fabric-producing process. By using such a thermoplastic resin (elongation thermoplastic resin), the mixed-fiber spunbonded nonwoven fabric obtained by combining with the continuous fibers of thermoplastic elastomer (A) and the nonwoven fabric laminate obtained by laminating the mixed-fiber spunbonded nonwoven fabrics exhibit a high bulkiness by being drawn and give comfortable touch feeling. In addition, the nonwoven fabric laminate can be imparted with a function of preventing excessive elongation. The maximum point elongation of the spunbonded nonwoven fabric comprising the thermoplastic resin (B) is not particularly limited, but is usually 300% or less.

Examples of the thermoplastic resins (B) include polyolefins, for example, homopolymers and copolymers of α-olefins (e.g., ethylene, propylene, 1-butene, 1-hexene, 4-methyl-1-pentene, and 1-octene), such as high-pressure low-density polyethylene, linear low-density polyethylene (so-called LLDPE), and high-density polyethylene (so-called HDPE), polypropylene (propylene homopolymer), polypropylene random copolymers, poly(1-butene), poly(4-methyl-1-pentene), ethylene/propylene random copolymers, ethylene/1-butene random copolymers, and propylene/1-butene random copolymers; polyesters (such as polyethylene terephthalate, polybutylene terephthalate, and polyethylene naphthalate); polyamides (such as nylon-6, nylon-66, and polymethaxyleneadipamide); polyvinyl chlorides, polyimides, ethylene/vinyl acetate copolymers, ethylene/vinyl alcohol copolymers, ethylene/(meth)acrylic acid copolymers, ethylene/acrylate/carbon monoxide copolymers, polyacrylonitriles, polycarbonates, polystyrenes, ionomers, and mixtures thereof. Among them, more preferred are high-pressure low-density polyethylene, linear low-density polyethylene (so called LLDPE), high-density polyethylene, propylene polymers such as polypropylene and polypropylene random copolymers, polyethylene terephthalate, and polyamides.

Among these thermoplastic resins (B), polyolefins are preferred, and propylene polymers are particularly preferred, from the viewpoints of spinning stability during the production and the stretching processability of a nonwoven fabric.

The propylene polymer is preferably a propylene homopolymer or a copolymer of propylene and significantly small amounts of one or more α-olefins having 2 or more (excluding 3) and preferably 2 to 8 (excluding 3) carbon atoms (such as ethylene, 1-butene, 1-pentene, 1-hexene, 1-octene, and 4-methyl-1-pentene) that have a melting point (Tm) of 155°C or higher and preferably in the range of 157°C to 165°C.

The melt flow rate (MFR: ASTM D-1238, 230°C, a load of 2160 g) of the propylene polymer is not particularly limited as long as melt spinning is possible, and is usually in the range of 1 to 1000 g/10 min, preferably 5 to 500 g/10 min, and more preferably 10 to 100 g/10 min. Furthermore, the propylene polymers according to the present invention usually have a ratio of the weight-average molecular weight (Mw) to the number-average molecular weight (Mn), Mw/Mn, in the range of 1.5 to 5.0. The ratio is preferably in the range of 1.5 to 3.0, in terms of satisfactory spinnability and giving fibers that are excellent particularly in fiber strength. The Mw and the Mn can be measured by GPC (gel permeation chromatography) according to a known process.

An olefin polymer composition including the propylene polymer and a small amount of HDPE can further enhance the drawing adaptability of a produced nonwoven fabric laminate and is therefore preferred. The amount of the HDPE to be added is preferably in the range of 1 to 20 wt%, more preferably 2 to 15 wt%, and further preferably 4 to 10 wt% based on the total amount (100 wt%) of the propylene polymer and the HDPE, from the viewpoints of the spinnability and the drawing processability.

The HDPE added to the propylene polymer is not particularly limited, but the density thereof is usually in the range of 0.94 to 0.97 $g/cm^3$, more preferably 0.95 to 0.97 $g/cm^3$, and further preferably 0.96 to 0.97 $g/cm^3$. In addition, the melt flow rate (MFR: ASTM D-1238, 190°C, a load of 2160 g) of the HDPE is not particularly limited as long as it is spinnable, but is usually in the range of 0.1 to 100 g/10 min, preferably 0.5 to 50 g/10 min, and more preferably 1 to 30 g/10 min, from the viewpoint of imparting elongatability. In the present invention, satisfactory spinnability means that the filaments are not broken and are not fused together when they are extruded from a spinning nozzle and are drawn.

Additives

In the present invention, the mixed-fiber spunbonded nonwoven fabric may further contain, for example, various stabilizers such as a heat stabilizer and a weather stabilizer, an antistatic agent, a slip agent, an antifogging agent, a lubricant, a dye, a pigment, a natural oil, a synthetic oil, or a wax, as optional components.

Examples of the stabilizers include anti-aging agents such as 2,6-di-t-butyl-4-methylphenol (BHT); phenolic antioxidants such as tetrakis[methylene-3-(3,5-di-t-butyl-4-hydroxyphenyl)propionate]methane, alkyl 6-(3,5-di-t-butyl-4-hydroxyphenyl)propionates, 2,2'-oxamidobis[ethyl-3-(3,5-di-t-butyl-4-hydroxyphenyl)]propionate, and Irganox 1010 (trade name, hindered phenolic antioxidant); metal salts of fatty acids such as zinc stearate, calcium stearate, and calcium 1,2-hydroxystearate; and fatty acid esters of polyhydric alcohols such as glycerin monostearate, glycerin distearate, pentaerythritol monostearate, pentaerythritol distearate, and pentaerythritol tristearate. These stabilizers may be used singly or in combination of two or more.

Mixed-fiber nonwoven fabric laminate

The mixed-fiber nonwoven fabric laminate of the present invention is a mixed-fiber nonwoven fabric laminate including at least two layers of mixed-fiber spunbonded nonwoven fabrics that are each obtained by combining the continuous fibers of thermoplastic elastomer (A) (hereinafter, also referred to as "thermoplastic elastomer (A) continuous fibers") and the continuous fibers of thermoplastic resin (B) (hereinafter, also referred to as "thermoplastic resin (B) continuous fibers") at fiber mixing ratios different from each other.

The term "fiber mixing ratio" represents the ratio of a specific kind of fiber contained in a nonwoven fabric layer made of a mixture of two or more kinds of fibers or the mixing ratios of various kinds of fibers in the nonwoven fabric layer. That is, "a fiber mixing ratio of the thermoplastic elastomer (A) continuous fibers" in a spunbonded nonwoven fabric layer comprising the thermoplastic elastomer (A) and the thermoplastic resin (B) is {[the weight of the thermoplastic elastomer (A) continuous fibers]/[the sum of the weight of the thermoplastic elastomer (A) continuous fibers and the weight of the thermoplastic resin (B) continuous fibers]}. Similarly, "a fiber mixing ratio of the thermoplastic resin (B) continuous fibers" is {[the weight of the thermoplastic resin (B) continuous fibers]/[the sum of the weight of the elastomer (A) continuous fibers and the weight of the thermoplastic resin (B) continuous fibers]}. Furthermore, the term "different fiber mixing ratios" in spunbonded nonwoven fabric layers each comprising the thermoplastic elastomer (A) and the thermoplastic resin (B) means that the mixing ratios of (A) and (B) in nonwoven fabric layers are different from each other.

In a mixed-fiber nonwoven fabric laminate including at least two layers, at least one layer thereof is desirably a mixed-fiber spunbonded nonwoven fabric layer (C-1) comprising preferably 40 to 95 wt%, more preferably 40 to 90 wt%, and further preferably 50 to 80 wt% of continuous fibers obtained from the thermoplastic elastomer (A) and 60 to 5 wt%, more preferably 60 to 10 wt%, and further preferably 50 to 20 wt% of the thermoplastic resin (B) continuous fibers (wherein, (A)+(B) = 100 wt%). In addition, another layer is desirably a mixed-fiber spunbonded nonwoven fabric layer (C-2) made of a fiber mixture of preferably 10 to 60 wt%, more preferably 10 to 55 wt%, and further preferably 10 to 50 wt% of continuous fibers obtained from the thermoplastic elastomer (A) and 90 to 40 wt%, more preferably 90 to 45 wt%, and further preferably 90 to 50 wt% of the thermoplastic resin (B) continuous fibers (wherein, (A)+(B) = 100 wt%).

A higher fiber mixing ratio of the thermoplastic elastomer (A) continuous fibers enhances the strechability, the flexibility, and so on of the resulting mixed-fiber spunbonded nonwoven fabric. On the other hand, a lower fiber mixing ratio of the thermoplastic elastomer (A) continuous fibers enhances the spinning stability and the formability, for example, the resulting mixed-fiber spunbonded nonwoven fabric is prevented from winding to a spinning apparatus (rollers) and is enhanced to wind to a nonwoven fabric-collecting belt.

Therefore, the mixed-fiber nonwoven fabric laminate of the present invention can simultaneously have high strechability and flexibility, comfortable touch feeling, and satisfactory formability by adjusting the fiber mixing ratio of each layer of the laminate according to a purpose. More specifically, for example, a mixed-fiber nonwoven fabric laminate provided with the above-mentioned properties in a balanced manner can be obtained by constituting the laminate with at least two layers including a mixed-fiber spunbonded nonwoven fabric layer (C-1) and a mixed-fiber spunbonded nonwoven fabric layer (C-2). In the mixed-fiber spunbonded nonwoven fabric layer (C-1), the fiber mixing ratio of the thermoplastic elastomer (A) continuous fibers is increased for obtaining strechability. In the mixed-fiber spunbonded nonwoven fabric layer (C-2), the fiber mixing ratio of the thermoplastic resin (B) continuous fibers is increased (the fiber mixing ratio of the thermoplastic resin (B) continuous fibers is preferably 40% or more from the viewpoints of flexibility and touch feeling) for obtaining comfortable touch feeling and flexibility or from the viewpoint of satisfactory formability. The (C-1) and the (C-2) may be successively laminated, or the (C-1) and the (C-2) may be laminated via another (mixed-fiber) spunbonded nonwoven fabric layer, a melt blown layer, a film layer, or an adhesive layer interposed therebetween.

Furthermore, another embodiment of the present invention is a nonwoven fabric laminate including at least three mixed-fiber spunbonded nonwoven fabric layers containing the continuous fibers of thermoplastic elastomer (A) and the continuous fibers of thermoplastic resin (B), wherein the three mixed-fiber spunbonded nonwoven fabric layers are laminated such that the fiber mixing ratio of the thermoplastic elastomer (A) in the mixed-fiber spunbonded nonwoven fabric layer (intermediate layer) positioned at the middle of the three mixed-fiber spunbonded nonwoven fabric layers is larger than those of the other two layers.

In addition, the other two mixed-fiber spunbonded nonwoven fabric layers on both sides of the intermediate layer may have the same or different fiber mixing ratios of the thermoplastic elastomer (A) continuous fibers. Furthermore, it is not necessary that the three mixed-fiber spunbonded layers are successively laminated. They may be laminated via a spunbonded nonwoven fabric layer, a melt blown layer, a film layer, or an adhesive layer interposed thereamong. Furthermore, additional mixed-fiber nonwoven fabric layer(s) may be laminated on either one or both outer sides of the three layers. The fiber mixing ratio of the thermoplastic elastomer (A) in the additional mixed-fiber nonwoven fabric layer (s) may be either larger or smaller than that of the intermediate layer.

The mixed-fiber nonwoven fabric laminate being flexible and having a surface with less sticky can be obtained by increasing the fiber mixing ratio of the thermoplastic elastomer (A) continuous fibers in the intermediate layer and reducing the fiber mixing ratios thereof in the two mixed-fiber spunbonded nonwoven fabric layers on both sides of the intermediate layer. Furthermore, the mixed-fiber nonwoven fabric laminate can have excellent curling resistance by adjusting the fiber mixing ratios in the two mixed-fiber spunbonded nonwoven fabric layers on both sides of the intermediate layer the same.

More specifically, the intermediate layer is a mixed-fiber spunbonded nonwoven fabric layer (D-1) comprising preferably 40 to 100 wt%, more preferably 40 to 95 wt%, and further preferably 50 to 90 wt% of the continuous fibers of thermoplastic elastomer (A) and 60 to 0 wt%, more preferably 60 to 5 wt%, and further preferably 50 to 10 wt% of the continuous fibers of thermoplastic resin (B) (wherein, (A)+(B) = 100 wt%).

In addition, one of the two mixed-fiber spunbonded nonwoven fabric layers on the both sides of the intermediate layer is a mixed-fiber spunbonded nonwoven fabric layer (D-2) comprising 10 to 60 wt%, more preferably 10 to 55 wt%, and further preferably 10 to 50 wt% of the continuous fibers of thermoplastic elastomer (A) and 90 to 40 wt%, more preferably 90 to 45 wt%, and further preferably 90 to 50 wt% of the continuous fibers of thermoplastic resin (B) (wherein, (A)+(B) = 100 wt%). The other of the two is a mixed-fiber spunbonded nonwoven fabric layer (D-3) comprising 10 to 60 wt%, more preferably 10 to 55 wt%, and further preferably 10 to 50 wt% of the continuous fibers of thermoplastic elastomer (A) and 90 to 40 wt%, more preferably 90 to 45 wt%, and further preferably 90 to 50 wt% of the continuous fibers of thermoplastic resin (B) (wherein, (A) + (B) = 100 wt%)

Furthermore, the above-described intermediate layer of the nonwoven fabric laminate is characterized in that the fiber mixing ratio of the thermoplastic elastomer (A) continuous fibers in (D-1) is larger than those in (D-2) and (D-3). when the layers constitute such a layer structure, a nonwoven fabric laminate excellent in flexibility and continuous formability (without adhering to a spinning apparatus) can be obtained due to the flexibility of the thermoplastic elastomer (A) in (D-1) and the continuous formability caused by non-adhesiveness of the thermoplastic resin (B) in (D-2) and (D-3).

The fiber mixing ratios of the thermoplastic elastomer (A) continuous fibers in the two mixed-fiber spunbonded nonwoven fabric layers (D-2) and (D-3) on the both sides of the intermediate layer (D-1) may be the same or different, but fiber mixing ratios that are the same or low in the difference can prevent curling after the lamination and enhance the formability and are therefore preferred. More specifically, when the difference in fiber mixing ratios of the thermoplastic elastomer (A) continuous fibers in (D-2) and (D-3) is controlled to preferably 40% or less, more preferably 30% or less, further preferably 20% or less, and particularly preferably in the range of 10 to 0% and the difference in mass per unit areas of (D-2) and (D-3) is controlled such that the values of (D-1)/(D-2) and (D-1)/(D-3) are each in the range of preferably 2 to 0.5, more preferably 1.5 to 0.67, further preferably 1.2 to 0.83, and particularly preferably 1.1 to 0.91, the mixed-fiber nonwoven fabric laminate is excellent in curling resistance and productivity and production efficiency thereof can be increased.

As described above, the quality and the formability of a mixed-fiber spunbonded nonwoven fabrics are modified depending on the fiber mixing ratio of the thermoplastic elastomer (A) continuous fibers. Therefore, in order to obtain a mixed-fiber nonwoven fabric laminate having high strechability and flexibility and satisfactory touch feeling and formability, the laminate is configured while controlling each of the fiber mixing ratios of the layers. More specifically, the laminate includes an intermediate layer (D-1) comprising only the thermoplastic elastomer (A) continuous fibers or comprising a nonwoven fabric layer whose fiber mixing ratio of the thermoplastic elastomer (A) continuous fibers is increased for imparting strechability and layers (D-2) and (D-3) having high fiber mixing ratios of the thermoplastic resin (B) continuous fibers as surfaces involving in formability of the laminate for imparting satisfactory touch feeling and flexibility. The fiber mixing ratios of the thermoplastic resin (B) continuous fibers in (D-2) and (D-3) herein are preferably 40 wt% or more from the viewpoints of the flexibility and the touch feeling.

Fiber diameters (average values) of the thermoplastic elastomer (A) continuous fibers and the thermoplastic resin (B) continuous fibers forming the mixed-fiber spunbonded nonwoven fabric according to the present invention are each usually in the range of 50 $\mu$m or less, preferably 5 to 40 $\mu$m, and more preferably 7 to 30 $\mu$m. The fiber diameters

of the thermoplastic elastomer (A) continuous fibers and the thermoplastic resin (B) continuous fibers may be the same or different.

The mass per unit area of the mixed-fiber nonwoven fabric laminate of the present invention can be determined according to various applications. For example, in the application to sanitary materials such as diapers, the mass per unit area is usually in the range of 200 g/m$^2$ or less, preferably 100 g/m$^2$ or less, more preferably 80 g/m$^2$ or less, and further preferably 60 to 15 g/m$^2$ as the total mass per unit area of the laminate from the viewpoints of the flexibility and the air permeability.

The mass per unit area of each mixed-fiber spunbonded nonwoven fabric constituting the mixed-fiber nonwoven fabric laminate also can be determined according to various applications, but, in terms of the strechability and the flexibility of a resulting mixed-fiber nonwoven fabric laminate, the mass per unit area of a mixed-fiber spunbonded layer having a high fiber mixing ratio of the thermoplastic elastomer (A) continuous fibers, for example, each mass per unit area of the above-mentioned (C-1) and (D-1) is usually in the range of 120 g/m$^2$ or less, preferably 80 g/m$^2$ or less, more preferably 50 g/m$^2$ or less, and further preferably 40 to 15 g/m$^2$. Similarly, the mass per unit area of a mixed-fiber spunbonded layer having a low fiber mixing ratio of the thermoplastic elastomer (A) continuous fibers, for example, each mass per unit area of the above-mentioned (C-2), (D-2), and (D-3) is usually in the range of 120 g/m$^2$ or less, preferably 80 g/m$^2$ or less, more preferably 50 g/m$^2$ or less, and further preferably 40 to 5 g/m$^2$.

When the mixed-fiber spunbonded nonwoven fabrics according to the present invention are laminated and integrated, various known confounding methods can be employed for the integration. When the lamination integration is performed off-line, winding without confounding may be performed, but slight pre-bonding by a known confounding method can improve the productivity. Examples of the confounding method include a process in which fibers are accumlated on a conveyer belt and then pressed with a nip roller. In this process, the roller is desirably heated for slight pre-bonding. Other examples of the pre-bonding include a process using means such as needle punching, water jetting, or ultrasound, a hot embossing process with an embossing roller, and a hot-air-through process. In any of these processes, confounding is preferably performed slighter than usual in terms of the texture after lamination and strechability. Such confounding methods may be employed alone or in a combination.

The mixed-fiber spunbonded nonwoven fabric according to the present invention can be produced by using the thermoplastic elastomer (A) and the thermoplastic resin (B) by a known method for producing a spunbonded nonwoven fabric, such as a method disclosed in Japanese Patent Laid-Open Publication No. 2004-244791.

Specifically, for example, first, the thermoplastic elastomer (A) and the thermoplastic resin (B) are each melt in respective different extruders. Subsequently, the melt polymers are each fed into respective different spinnerets (dies) having a large number of spinning holes (nozzles). The thermoplastic elastomer (A) and the thermoplastic resin (B) are simultaneously extruded from the respective spinning holes independently, and then the melt-spun continuous fibers of thermoplastic elastomer (A) and thermoplastic resin (B) are fed into a cooling chamber. After the cooling with cooling wind in the cooling chamber, the continuous fibers are drawn (towed) with drawing air and accumlated on a conveying collection surface to produce a mixed-fiber spunbonded nonwoven fabric of the present invention. The melt temperature of each polymer is not particularly limited as long as it is of not lower than the softening or melting temperature and of lower than the decomposition temperature of the polymer. The melt temperature can be determined depending on the polymer used and the like. The spinneret temperature depends on the polymer used. For example, in the case that the thermoplastic elastomer (A) is a thermoplastic polyurethane elastomer or an olefin copolymer elastomer and the thermoplastic resin (B) is a propylene polymer or an olefin polymer composition of a propylene polymer and HDPE, the spinneret temperature can be set in the range of usually 180°C to 240°C, preferably 190°C to 230°C, and more preferably 200°C to 225°C.

The temperature of the cooling wind is not particularly limited as long as the polymer is solidified, and is usually in the range of 5°C to 50°C, preferably 10°C to 40°C, and more preferably 15°C to 30°C. The velocity of the drawing air is usually in the range of 100 to 10,000 m/min and preferably 500 to 10,000 m/min.

The mixed-fiber nonwoven fabric laminate of the present invention may be produced by separately preparing mixed-fiber spunbonded nonwoven fabrics containing the thermoplastic elastomer (A) continuous fibers at different fiber mixing ratios by the above-described process and then laminating them. As the method of lamination, for example, the mixed-fiber spunbonded nonwoven fabrics including the thermoplastic elastomer (A) continuous fibers at different fiber mixing ratios from each other may be successively laminated with a spunbonded nonwoven fabric-producing apparatus having at least two lines for spinning.

The mixed-fiber spunbonded nonwoven fabrics according to the present invention or the mixed-fiber nonwoven fabric laminates of the present invention can be integrated by various known confounding methods according to the applications. In the confounding methods, after a plurality of mixed-fiber spunbonded layers including the thermoplastic elastomer (A) continuous fibers at different fiber mixing ratios from each other are stacked, for example, a process using means such as needle punching, water jetting, or ultrasound, a hot embossing process with an embossing roller, or a hot-air-through process is employed. By using such a process, the mixed-fiber spunbonded nonwoven fabrics and the mixed-fiber nonwoven fabric laminates can be integrated. In the integration of the mixed-fiber spunbonded nonwoven fabrics and the mixed-fiber nonwoven fabric laminates, such confounding methods may be employed alone or in a combination. Furthermore, the method of stacking the plurality of layers to be applied may be, for example, a process of stacking, on off-line, a plurality of layers having different fiber mixing ratios of the thermoplastic elastomer (A) continuous fibers provided with slight pre-bonding by various known confounding methods, a process of continuously stacking mixed-fiber spunbonded nonwoven fabric layers having different fiber mixing ratios of the thermoplastic elastomer (A) continuous fibers with a spinning apparatus having a plurality of lines, or a process of a combination of the process of off-line and the process of continuous stacking.

In the case of heat sealing by hot embossing, the embossed area ratio of the mixed-fiber spunbonded nonwoven fabric according to the present invention is usually 5% to 20% and preferably 5% to 10%, and the non-embossed unit area is in the range of 0.5 mm$^2$ or more and preferably 4 to 40 mm$^2$. The term "non-embossed unit area" means, in a non-embossed portion of a minimum unit surrounded on all four directions by embossed portions, the maximum area of a quadrangle inscribed in an embossed area. Examples of stamped shapes include circles, ellipsoids, ellipses, squares, lozenges, rectangles, tetragons, and successive shapes of these basic shapes. By embossing so as to satisfy the above-mentioned embossed area ratio and non-embossed unit area, bonding points are formed in the embossed portions to substantially bond between the thermoplastic elastomer (A) continuous fibers and the thermoplastic resin (B) continuous fibers constituting the mixed-fiber spunbonded nonwoven fabric and between the mixed-fiber spunbonded nonwoven fabric layers. In addition, in the portions surrounded by enbossed portions of the mixed-fiber spunbonded nonwoven fabric layer, the thermoplastic elastomer (A) continuous fibers having elasticity and the thermoplastic elastomer (B) continuous fibers (elongation fibers) having substantially less elasticity than that of the thermoplastic elastomer (A) continuous fibers are present with a high degree of freedom. In the mixed-fiber spunbonded nonwoven fabric having such a structure, the residual strain is decreased and satisfactory strechability is exhibited after a drawing process.

A larger embossed area ratio reduces the range of drawable area, but increases stress. A smaller embossed area ratio increases the range of drawable area, but increases the embossing pitch and has a tendency of slightly increasing residual stain.

The nonwoven fabric laminate of the present invention may be further applied to a drawing process. Furthermore, the nonwoven fabric laminate before the drawing may be applied to confounding by the above-mentioned confounding method, preferably by embossing.

The nonwoven fabric laminate of the present invention has a difference in elongation recovery ratios between the continuous fibers of thermoplastic elastomer (A) and the continuous fibers of thermoplastic resin (B) constituting the mixed-fiber spunbonded nonwoven fabric layer. Therefore, after the drawing, the drawn continuous fibers of thermoplastic elastomer (A) elastically recover to a length similar to that before the drawing, whereas the length of the continuous fibers of thermoplastic resin (B) remains in the drawn state. Consequently, the continuous fibers of thermoplastic resin (B) are folded on the surface of the nonwoven fabric laminate to provide a more bulky and flexible nonwoven fabric laminate.

In addition, the mixed-fiber nonwoven fabric laminate of the present invention may be laminated with additional layer according to various applications.

Examples of the additional layer laminated to the mixed-fiber nonwoven fabric laminate of the present invention include a knitted fabric, a woven fabric, a nonwoven fabric, and a film. In the case that the nonwoven fabric laminate of the present invention and the additional layer are laminated, various known methods represented by hot embossing, heat sealing such as ultrasound sealing, mechanical confounding such as needle punching or water jetting, a process using a hot-melt adhesive, a urethane adhesive, or the like, and extrusion lamination can be used for laminating the nonwoven fabric laminate of the present invention and the additional layer.

The nonwoven fabric laminated to the nonwoven fabric laminate of the present invention may be a known nonwoven fabric, and examples thereof include spunbonded nonwoven fabrics, melt blown nonwoven fabrics, wet-process nonwoven fabrics, dry-process nonwoven fabrics, dry-process nonwoven pulp fabrics, flash spun nonwoven fabrics, and opening nonwoven fabrics.

Examples of the film laminated to the nonwoven fabric laminate of the present invention include moisture-permeable films and air-permeable films.

Moisture-permeable film

The moisture-permeable film constituting the nonwoven fabric laminate of the present invention is a film comprising a thermoplastic elastomer (referred to as thermoplastic elastomer (A') in order to distinguish from thermoplastic elastomer (A) contained in a mixed-fiber spunbonded nonwoven fabric layer). The thermoplastic elastomer (A') is a film usually having a water vapor transmission rate of 2000 g/m$^2$·day or more, preferably 3000 g/m$^2$·day, and further preferably 4000 g/m$^2$·day or more as a moisture permeability at a thickness of 30 $\mu$m measured by a JIS L1099 A-1 method (40°C, relative humidity: 90%, conditions for a CaCl$_2$ method).

The moisture-permeable film according to the present invention may be properly selected according to various applications, but the thickness is usually in the range of 10 to 50 μm and preferably 15 to 40 μm. The thickness is desirably 10 μm or more from the viewpoints of preventing pinholes during the lamination with a nonwoven fabric and maintaining the mechanical strength and proper water resistance and is desirebly 50 μm or less from the viewpoints of obtaining satisfactory moisture permeability and flexibility.

The moisture-permeable film according to the present invention is formed using the thermoplastic elastomer (A') by a known film-forming process, for example, by melting the thermoplastic elastomer (A') in an extruder and forming it into a film with a T-die or a cyclic die. Since a moisture-permeable elastomer is generally much sticky and causes blocking, the film may be formed directly or indirectly on the mixed-fiber spunbonded nonwoven fabric layer by extrusion as a film layer.

Thermoplastic elastomer (A')

The thermoplastic elastomer (A') according to the present invention may be any thermoplastic elastomer that has moisture permeability when it is formed into a film. The moisture permeability can be confirmed by, for example, a JIS L1099 A-1 method (40°C, relative humidity: 90%, conditions for a $CaCl_2$ method) in order to recognize water vapor permeability. In this case, preferably, a film 30 μm in thickness having a water vapor transmission rate, when measured by the JIS L1099 A-1 method, of 2000 $g/m^2 \cdot day$ or more, preferably 3000 $g/m^2 \cdot day$, is preferably used in the present invention.

Preferred examples of the thermoplastic elastomer (A') include polyester elastomers (A'-1), polyamide elastomers (A'-2), and thermoplastic polyurethane elastomers (A'-3).

The thermoplastic elastomer (A') can be blended with additives that are usually used, such as an antioxidant, a weather stabilizer, an antistatic agent, an antifogging agent, a blocking-preventing agent, a lubricant, a nucleating agent, or a pigment, or another polymer according to needs within ranges that do not impair the effects of the present invention.

Polyester elastomer (A'-1)

Examples of the polyester elastomer (A'-1) include block copolymers obtained by block-copolymerizing a hard segment derived from an aromatic polyester having a structure unit represented by the following formula (I) and a soft segment derived from an aliphatic polyether having a structure unit represented by the following formula (II).

$$-O-D-O-CO-R-CO- \qquad (I)$$

$$-O-G-O-CO-R-CO- \qquad (II)$$

In the formulae above, D denotes a divalent residue resulted from removeing two hydroxyl groups from a diol having a molecular weight of about 250 or less, R denotes a divalent residue resulted from removing two carboxyl groups from a dicarboxylic acid having a molecular weight of about 300 or less, and G denotes a divalent residue resulted from removing hydroxyl groups on both ends from a poly(alkylene oxide)glycol having an average molecular weight of about 400 to about 3500. Here, the amount of the ethylene oxide group that is inserted into the structure unit represented by formula (II), i.e., copolyether ester of poly(alkylene oxide)glycol, is usually about 25 to 68 mass% to the total mass of the copolyether ester.

In the present invention, it is particularly preferred that the aromatic polyester is tetramethylene terephthalate and the aliphatic polyether is alkylene ether glycol, and specific examples thereof include polybutylene terephthalate/polytetramethylene ether glycol block copolymers. Concrete commercially available products are manufactured and marketed under trade names such as HYTREL (trade name, manufactured by E.I. DuPont & Company) and Pelprene (trade name, manufactured by Toyobo Co., Ltd.).

Polyamide elastomer (A'-2)

Examples of polyamide elastomer (A'-2) include multiblock copolymers obtained by using a polyamide for hard segment and using a polyester or polyol diol with a low glass transition temperature for a soft segment. Examples of the polyamide component herein include nylon 6, nylon 66, nylon 610, nylon 11, and nylon 12. Among these polyamides, nylon 6 and nylon 12 are preferred. Examples of the polyether diol include poly(oxytetramethylene)glycol and poly(oxypropylene)glycol. Examples of the polyester diol include poly(ethylene/1,4-adipate)glycol, poly(butylene/1,4-adipate)glycol, and polytetramethylene glycol. Specific examples of polyamide elastomer (A'-2) include nylon 12/polytetramethylene glycol block copolymers. Concrete commercially available polyamide elastomers are manufactured and marketed under trade names such as Daiamide (manufactured by Daicel Huls Ltd.) and PEBAX (manufactured by AtoChem Co., Ltd.) (they are trade names).

Thermoplastic polyurethane elastomer (A'-3)

Examples of the thermoplastic polyurethane elastomer (A'-3) include block copolymers comprising a hard segment of polyurethane prepared by reaction of short chain polyol (molecular weight: 60 to 600) and diisocyanate and a soft segment of polyurethane prepared by reaction of long chain polyol (molecular weight: 600 to 4000) and diisocyanate. Examples of the diisocyanate include toluene diisocyanate and diphenylmethane diisocyanate, and examples of the short chain polyol include ethylene glycol, 1,3-propylene glycol, and bisphenol A.

Examples of the thermoplastic polyurethane elastomer (A'-3) include polymers (polyether polyurethane) prepared by addition polymerization of polylactone ester polyol such as polycaprolactone glycol with diisocyanate in the presence of short chain polyol; polymers (polyester polyurethane) prepared by addition polymerization of adipic acid ester polyol such as poly(ethylene/1,4-adipate)glycol or poly(butylene/1,4-adipate)glycol with diisocyanate in the presence of short chain polyol; and polymers prepared by addition polymerization of polytetramethylene glycol obtained by ring-opening of tetrahydrofuran with diisocyanate in the presence of short chain polyol. Concrete commercially available thermoplastic polyurethane elastomers are manufactured and marketed under trade names such as Volkolan (manufactured by Bayer AG), Chemigum SL (manufactured by Goodyear Co.), Adiprene (manufactured by DuPont & Company), and Valcaprene (manufactured by I.C.I. Ltd.) (they are trade names).

Among these elastomers, the polyester elastomers (A'-1) and the thermoplastic polyurethane elastomer (A'-3) are preferred in terms of excellent moisture permeability. In particular, the thermoplastic polyurethane elastomers (A'-3) further have strechability and therefore are particularly preferred.

Air-permeable film

The air-permeable film according to the present invention comprises a thermoplastic resin (also referred to as thermoplastic resin (B') in order to distinguish from the thermoplastic resin (B)) and is a film that permeates gas such as oxygen and water vapor but hardly permeates liquid such as water. The film has micropores such that the moisture permeability determined by a JIS-L1099 A-1 method (40°C, relative humidity: 90%, conditions for a $CaCl_2$ method) is usually 1000 to 20000 $g/m^2 \cdot 24$ hrs.

The moisture permeability of a (precursor) film not yet imparted with air permeability may be approximately 0 $g/m^2 \cdot 24$ hrs, as long as the film is imparted with a moisture permeability within the above-mentioned range as an air-permeable film by being laminated to the above-mentioned mixed-fiber spunbonded nonwoven fabric to form a nonwoven fabric laminate and then being applied to a suitable process.

The air-permeable film according to the present invention is a thermoplastic resin composition containing the thermoplastic resin (B'), preferably, the thermoplastic resin (B') and a filler. Furthermore, when the air-permeable film according to the present invention is a resin composition containing the thermoplastic resin (B') and a filler, the blending amount of the filler is more preferably 30 to 75 wt%, further more preferably 40 to 70 wt%, and particularly preferably 40 to 60 wt% (wherein, the total amount of thermoplastic resin (B') and the filler is 100 wt%). The air-permeable film according to the present invention can be obtained by extruding the thermoplastic resin composition into a (precursor) film and drawing the film in at least one direction.

The filler is preferably an organic compound filler, for example, crosslinked particles such as polymethylmethacrylate (PMMA) particles or polystyrene particles or a resin that is incompatible with the thermoplastic resin used as a film base material (for example, polyethylene terephthalate or polyamide when a polyolefin resin is used); or an inorganic compound filler such as calcium carbonate, barium sulfate, calcium sulfate, barium carbonate, magnesium hydroxide, aluminum hydroxide, zinc oxide, magnesium oxide, titanium oxide, silica, or talc.

Preferable inorganic compound fillers are calcium carbonate and barium sulfate particles having an average particle diameter in the range of 10 $\mu$m or less, in particular, 0.5 to 5 $\mu$m.

Furthermore, most preferable inorganic compound fillers are those of which inorganic compound has surfaces treated with higher fatty acid such as stearic acid or lauric acid or a metal salt thereof in order to increase dispersibility with a thermoplastic resin as a base material.

Various known thermoplastic resins can be used as the thermoplastic resin (B') to be used in the air-permeable film, and polyolefin resins such as polyethylene, polypropylene, and poly(1-butene) are excellent in water resistance and are therefore preferred. In particular, linear low-density polyethylenes, namely, random copolymers which have a density in the range of 0.900 to 0.940 $g/cm^3$ and comprise ethylene and $\alpha$-olefin having 3 or more carbon atoms, preferably 4 to 10 carbon atoms, such as propylene, 1-butene, 1-heptene, 1-hexene, 1-octene, 4-methyl-1-pentene, or 1-decene, can provide an air-permeable film having excellent flexibility and are therefore preferred.

The ethylene/$\alpha$-olefin random copolymer is not particularly limited and may be one that is prepared using various known catalysts such as a Ziegler catalyst or a methallocene catalyst. Ethylene/$\alpha$-olefin random copolymers prepared using a methallocene catalyst can provide an air-permeable film having excellent mechanical strength such as tearing strength and are therefore preferred.

A small amount of a high-pressure low-density polyethylene may be added to the ethylene/$\alpha$-olefin random copolymer in order to enhance the drawability, and a small amount of polypropylene may be added in order to increase the tensile strength of the resulting air-permeable film.

The air-permeable film according to the present invention can be properly selected according to various applications, and usually has a thickness in the range of 10 50 g/m$^2$, preferably 12 to 40g/m$^2$, and further preferably 15 to 30 g/m$^2$. The thickness is desirably 10 g/m$^2$ or more from the viewpoints of ensuring mechanical strength of the air-permeable film and providing water resistance, and is preferably less than 50 g/m$^2$ from the viewpoint of ensuring sufficient air permeability.

EXAMPLES

The present invention will be further specifically described based on Examples below, but is not limited to these Examples.

Physical property values and so on in Examples and Comparative Examples were measured by the following methods.

(1) Mass per unit area (g/m$^2$)

Six test pieces of 200 mm (MD) and 50 mm (CD) in size were sampled from a nonwoven fabric and/or a nonwoven fabric laminate. The sampling positions of the MD and the CD were each at three arbitrary spots (six spots in total). Then, the mass (g) of each test piece sampled was measured with an electronic balance scale (manufactured by Kensei Kogyo Co., Ltd.), and the average mass value of the test pieces was determined. The mass (g) per square meter was calculated from the average and rounded to one decimal place to give the mass per unit area (g/m$^2$) of each nonwoven fabric sample.

(2) Maximum elongation (%)

Six test pieces of 200 mm (MD) and 50 mm (CD) in size were sampled from a nonwoven fabric and/or a nonwoven fabric laminate. The sampling positions of the MD and the CD were each at three arbitrary spots (six spots in total). Then, each test piece sampled was applied to a tensile test with a universal tensile tester (manufactured by Intesco Co., Ltd., model: IM-201) under conditions of a spun distance 10 of 100 mm and a tensile rate of 100 mm/min, and the elongation (maximum elongation (%)) at the maximum load was determined. The maximum elongation was obtained by calculating the average from the values of the six spots (three spots for each MD and CD) and rounding the average to one decimal place.

(3) Residual strain (%)

Six test pieces of 200 mm (MD) and 50 mm (CD) in size were sampled from a nonwoven fabric and/or a nonwoven fabric laminate. The sampling positions of the MD and the CD were each at three arbitrary spots (six spots in total). Then, each test piece sampled was drawn under conditions of a distance between chucks of 100 mm, a tensile rate of 100 mm/min, and a drawing ratio of 100% with a universal tensile tester (manufactured by Intesco Co., Ltd., model: IM-201) and then immediately recovered to the original length at the same rate. The strain at the time of recovery was measured as the residual strain (%). The residual strain was determined by calculating the average from the values at the six spots (three spots for each MD and CD) and rounding the average to one decimal place.

(4) Curling

Test pieces of 200 mm (MD) and 50 mm (CD) in size were sampled from a nonwoven fabric and/or a nonwoven fabric laminate. Each test piece sampled was visually observed for curling in the case that the test piece was placed on a table so that the front surface thereof is upper surface and in the case that the test piece was placed on a table so that the back surface thereof is upper surface. When the test piece had a portion where the nonwoven fabric was spaced from the table in either case of the front or back surface, the test piece was determined to have "occurrence" of curling, and when there was no portion being spaced from the table, it was determined to be "none" of curling.

(5) Stickiness

Ten panelists touched a nonwoven fabric and/or a nonwoven fabric laminate with their hands and evaluated the stickiness according to the following criteria. Here, the condition before the measurement of "residual strain in the above (3)" was evaluated as "before drawing treatment", and the condition after the measurement was evaluated as "after drawing treatment".

◎: All 10 panelists felt pleasant touch feeling without stickiness.

○: From 9 to 7 panelists in 10 panelists felt pleasant touch feeling without stickiness.

△: From 6 to 3 panelists in 10 panelists felt pleasant touch feeling without stickiness.

X: From 2 to 0 panelists in 10 panelists felt pleasant touch feeling without stickiness.

(6) Fuzzing

Adhesiveness of a nonwoven fabric or fibers to an embossing roller during integration of nonwoven fabrics and/or nonwoven fabric laminates by hot embossing, a subsequent spinning apparatus, or the like was visually observed. When adhesiveness of a nonwoven fabric or fibers was observed, it was determined to have "occurrence" of fuzzing, and when adhesiveness was not observed, it was determined to be "none" of fuzzing.

The thermoplastic polyurethane elastomer (TPU) used in Examples and Comparative Examples was analyzed and evaluated by the following methods.

(7) Solidification starting point

Measurement was performed with a differential scanning calorimeter (DSC220C) connected to SSC5200H Disk Station manufactured by Seiko Instruments Inc. A sample was prepared by placing about 8 mg of pulverized TPU in an aluminum pan and applying a cover to the pan for crimping. Similarly, aluminum was placed as a reference. The sample and the reference were each set at a predetermined position in a cell and were applied to measurement under a nitrogen gas flow at a flow rate of 40 Nml/min. The sample and the reference were heated from room temperature to 230°C at a rate of 10°C/min, held at this temperature for 5 minutes, and then cooled to -75°C at a rate of 10°C/min. The starting temperature of an exothermic peak attributed to the solidification of TPU recorded at this time was measured as the solidification starting point (unit: °C).

(8) Number of polar solvent-insoluble particles

The measurement was performed using Multisizer II manufactured by Beckman Coulter, Inc. as a particle size distribution analyzer based on a pore electric resistance method. Dimethylacetamide (3500 g, special grade, manufactured by Wako Pure Chemical Industries, Ltd.) and ammonium thiocyanate (145.83 g, special grade, manufactured by Junsei Chemical Co., Ltd.) were weighed in 5-L separable flask and were dissolved at room temperature over 24 hours.

Subsequently, reduced pressure filtration through a membrane filter of 1 $\mu$m pore size was performed to give reagent A. Reagent A (180 g) and TPU pellets (2.37 g) were precisely weighed in 200 cc glass bottle, and soluble part in the TPU was dissolved over 3 hours to give a measurement sample. The Multisizer II was equipped with an aperture tube of 100 $\mu$m, and the solvent in the apparatus was replaced with reagent A, and then the degree of pressure reduction was adjusted to about 3000 mmAq. One hundred and twenty grams of reagent A were weighed in a sufficiently washed beaker for sample collection to confirm that the pulse amount generated in blank measurement was 50 pulses per minute or less. Current value and Gain were optimized according to the manual, and then calibration was performed using 10 $\mu$m of uncrosslinked polystyrene standard particles. The measurement was performed for 210 seconds using 120 g of reagent A and about 10 g of a measurement sample weighed in a sufficiently washed beaker for sample collection. The number of particles counted by this method was divided by the weight of TPU suctioned in the aperture tube, and the quotient was used as the number of polar solvent-insoluble particles (unit: the number of particles per gram) in the TPU. The weight of the TPU was calculated by the following equation.

$$\text{TPU weight} = \{(A/100) \times B/(B+C)\} \times D,$$

in the equation, A: TPU concentration (wt%) in a measurement sample, B: weight (g) of the measurement sample weighed in a beaker, C: weight (g) of reagent A weighed in the beaker, and D: solution amount (g) suctioned in the aperture tube during measurement (210 seconds).

(9) Ratio of heat of fusion of hard domain

The measurement was performed with a differential scanning calorimeter (DSC220C) connected to SSC5200H Disk Station manufactured by Seiko Instruments Inc. A sample was prepared by placing about 8 mg of pulverized TPU in an aluminum pan and applying a cover to the pan for crimping. Similarly, aluminum was placed as a reference. The sample and the reference were each set at a predetermined position in a cell and were applied to measurement under a nitrogen gas flow at a flow rate of 40 Nml/min. The sample and the reference were heated from room temperature to 230°C at a rate of 10°C/min. At this point, the total (a) of heat of fusion determined from endothermic peaks within the peak temperature range of 90°C to 140°C and the total (b) of heat of fusion determined from endothermic peaks within the peak temperature range of higher than 140°C to 220°C were determined, and the ratio (unit: %) of heat of fusion of a hard domain was determined by the following equation:
```
```

$$\text{Ratio (\%) of heat of fusion of hard domain} = a/(a+b)\times100.$$

(10) Melt viscosity at 200°C (hereinafter, simply referred to as "melt viscosity")

The melt viscosity (unit: unit: Pa·s) of TPU at 200°C was measured with a Capirograph (manufactured by Toyo Seiki K.K., model: 1C) at a shear rate of 100 sec$^{-1}$ using a nozzle with a length of 30 mm and a diameter of 1 mm.

(11) Moisture value of TPU

The moisture value (unit: ppm) of TPU was measured using a combination of a water content measurement device (AVQ-5S, manufactured by Hiranuma Sangyo Corp.) and an evaporator (EV-6, manufactured by Hiranuma Sangyo Corp.). Approximately 2 g of TPU pellets weighed on a sample-heating plate were charged into an oven at 250°C. The evaporated water was led to a titration cell of the water content measurement device in which residual moisture was removed in advance, and titrated using a Karl Fischer reagent. When the change in the voltage between titration electrodes, which is caused by a change in water content in the cell, was not observed for 20 seconds, it was determined that the titration was terminated.

(12) Hardness of Shore A

The hardness of TPU was measured in accordance with a method as described in JIS K-7311 at 23°C and a relative humidity of 50% with a durometer Type A.

TPU Production Example 1

Diphenylmethane diisocyanate (hereinafter, referred to as MDI) was charged in tank A under a nitrogen atmosphere and adjusted to 45°C while stirring in such a manner that an air bubbles is not included.
Tank B was charged with 628.6 parts by weight of polyester polyol (manufactured by Mitsui Takeda Chemicals, Inc., trade name: Takelac U2024) having a number-average molecular weight of 2000, 2.21 parts by weight of Irganox 1010, and 77.5 parts by weight of 1,4-butane diol under a nitrogen atmosphere and were adjusted to 95°C while stirring. Here, this mixture is referred to as polyol solution 1.
The amount of the hard segment calculated from these reaction materials was 37.1 wt%.
Then, MDI and polyol solution 1 were quantitatively passed through a high-speed agitator (SM40) adjusted to 120°C at flow rates of 17.6 kg/h and 42.4 kg/h, respectively, by a liquid-transporting line via a gear pump and a current meter and were stirred for mixing at 2000 rpm for 2 minutes and then were passed through static mixers. The static mixer unit included first to third static mixers (temperature: 230°C) comprising connected three static mixers having a tube length of 0.5 m and an inner diameter of 20 mmΦ, fourth to sixth static mixers (temperature: 220°C) comprising connected three static mixers having a tube length of 0.5 m and an inner diameter of 20 mmΦ, seventh to twelfth static mixers (temperature: 210°C) comprising connected six static mixers having a tube length of 1.0 m and an inner diameter of 34 mmΦ, and thirteenth to fifteenth static mixers (temperature: 200°C) comprising connected three static mixers having a tube length of 0.5 m and an inner diameter of 38 mmΦ, all of which are connected in series.

The reaction product discharged from the fifteenth static mixer was pressed into a single-screw extruder (diameter: 65 mm, temperature: 180°C to 210°C) having a polymer filter (manufactured by Nagase & Co., Ltd., trade name: Dena Filter) at the top end via the gear pump and was extruded from a strand die.

The reaction product was cooled with water and then continuously pelletized with a pelletizer. Subsequently, the resulting pellets were charged in a drier and dried at 100°C for 8 hours to give a thermoplastic polyurethane elastomer with a water value of 40 ppm. This thermoplastic polyurethane elastomer was continuously extruded with a single-screw extruder (diameter: 50 mm, temperature: 180°C to 210°C) and pelletized. Again, the pellets were dried at 100°C for 7 hours to give thermoplastic polyurethane elastomer (A-1) having a water value of 57 ppm.

In A-1, the solidification starting point was 103.7°C, the number of polar solvent-insoluble particles was 1500000 particles per gram, the hardness of a test piece prepared by extrusion was 86 A, the melt viscosity at 200°C was 1900 Pa·s, and the ratio of heat of fusion of the hard domain was 35.2%.

Example 1

Preparation of thermoplastic resin composition for spunbonded nonwoven fabric

Mixed were 96 wt% of propylene homopolymer having an MFR (measured in accordance with ASTM D1238 at temprature of 230°C with a load of 2.16 kg) of 60 g/10 min, a density of 0.91 g/cm$^3$, a melting point of 160°C, and Mw/Mn of 2.9 (hereinafter, abbreviated to "PP-1") and 4 wt% of a high-density polyethylene having an MFR (measured in accordance with ASTM D1238 at temperature of 190°C with a load of 2.16 kg) of 5 g/10 min, a density of 0.97 g/cm$^3$, and a melting point of 134°C (hereinafter, abbreviated to "HDPE") to prepare thermoplastic resin composition (B-1).

Production of mixed-fiber nonwoven fabric laminate Production of mixed-fiber spunbonded nonwoven fabric (C-1-1)

The above-mentioned thermoplastic polyurethane elastomer (A-1) and thermoplastic resin composition (B-1) were each independently melt in an extruder having a diameter of 75 mmΦ and an extruder having a diameter of 50 mmΦ, respectively. A-1 and B-1 were then melt-spun with a spunbonded nonwoven fabric-forming machine (the length in the direction orthogonal to the machine flow direction on a collection surface: 800 mm) having a spinneret by a spunbonding method under conditions that the temperatures of both the resin and the die were 210°C, the cooling wind temperature was 20°C, and the drawing air rate was 3750 m/min. Thus, a web comprising continuous fibers A comprising A-1 and continuous fibers B conprising B-1 was accumulated at a fiber mixing ratio of 60:40 (weight ratio) on the collection surface. The spinneret had a nozzle pattern in which discharge apertures for A-1 and discharge apertures for B-1 are alternately arranged. The diameter of the nozzle for A-1 (fibers A) was 0.75 mmΦ, and the diameter of the nozzle for B-1 (fibers B) was 0.6 mmΦ. The nozzle pitches were 8 mm in the longitudinal direction and 11 mm in the width direction. The ratio of the numbers of nozzles for fibers A and nozzles for fibers B was 1:1.45. The discharging amount by a single aperture was adjusted to 1.1 g/(min·aperture) for fibers A and 0.5 g/(min·aperture) for fibers B.

The accumlated web comprising the mixed continuous fibers was formed into mixed-fiber spunbonded nonwoven fabric (C-1-1) with nip rollers (linear pressure: 10 kg/cm) which was placed on a belt and was coated with a non-adhesive material. The mass per unit area of the resulting mixed-fiber spunbonded nonwoven fabric was 15 g/m$^2$. In addition, regarding the fiber diameters of the accumlated web comprising the mixed continuous fibers, the larger fiber diameter, i.e., the diameter of fibers A was 35.2 μm, and the smaller fiber diameter, i.e., the diameter of fibers B was 17.0 μm.

Production of mixed-fiber spunbonded nonwoven fabric (C-2-1)

Mixed-fiber spunbonded nonwoven fabric (C-2-1) comprising continuous fibers A and continuous fibers B at a fiber mixing ratio of 20:80 (weight ratio) and having a mass per unit area of 15 g/m$^2$ was produced by spinning and accumulation under the same conditions as those in Example 1 except that the discharging amount by a single aperture was adjusted to 0.4 g/(min·aperture) for fibers A (A-1) and 1.0 g/(min·aperture) for fibers B (B-1).

Production of mixed-fiber nonwoven fabric laminate

Mixed-fiber spunbonded nonwoven fabric layers (C-1-1) and (C-2-1) were stacked and applied to lamination integration by embossing under the following conditions. The conditions for embossing were as follows: The (C-1-1) layer side was flat-rolled in the condition that the heating temperature was 100°C and a linear pressure was 30 kg/cm. The (C-2-1) layer side was embossed-rolled in the conditiion that the heating temperature was 120°C and a linear pressure was 30 kg/cm, so as to have a pattern with an area ratio of 18% and a stamped area of 0.41 mm$^2$.

The resulting nonwoven fabric laminate was evaluated by the above-described methods. Table 1 shows the evaluation results.

Example 2

Production of mixed-fiber spunbonded nonwoven fabric (D-1-1)

Mixed-fiber spunbonded nonwoven fabric (D-1-1) comprising the continuous fibers A and the continuous fibers B at a fiber mixing ratio of 70:30 (weight ratio) and having a mass per unit area of 10 g/m$^2$ was produced in accordance with the method as described in Example 1.

Production of mixed-fiber spunbonded nonwoven fabrics (D-2-1) and (D-3-1)

Mixed-fiber spunbonded nonwoven fabrics (D-2-1) and (D-3-1) comprising the continuous fibers A and the continuous fibers B at a fiber mixing ratio of 41:59 (weight ratio) and having a mass per unit area of 10 g/m$^2$ were produced in accordance with the method as described in Example 1.

Production of mixed-fiber nonwoven fabric laminate

Mixed-fiber nonwoven fabric layer (D-1-1) was arranged between (D-2-1) and (D-3-1) to laminate three layers in total, and lamination integration by embossing were performed under the following conditions to give a mixed-fiber nonwoven fabric laminate having three layers. The conditions for embossing were as follows: The flat-rolling and the embossed-rolling of a pattern with an area ratio of 18% and a stamped area of 0.41 mm$^2$ were conducted at a heating temperature of 110°C at a linear pressure of 30 kg/cm.
The resulting mixed-fiber nonwoven fabric laminate was evaluated by the above-described methods. Table 2 shows the evaluation results.

Example 3

Production of mixed-fiber spunbonded nonwoven fabric (D-1-2)

Mixed-fiber spunbonded nonwoven fabric (D-1-2) comprising continuous fibers A and continuous fibers B at a fiber mixing ratio of 50:50 (weight ratio) and having a mass per unit area of 20 g/m$^2$ was produced in accordance with the method as described in Example 1.

Production of mixed-fiber spunbonded nonwoven fabrics (D-2-2) and (D-3-2)

Mixed-fiber spunbonded nonwoven fabrics (D-2-2) and (D-3-2) each comprising the continuous fibers A and the continuous fibers B at a fiber mixing ratio of 30:70 (weight ratio) and having a mass per unit area of 5 g/m$^2$ were produced in accordance with the method as described in Example 1.

Production of mixed-fiber nonwoven fabric laminate

Mixed-fiber nonwoven fabric layer (D-1-2) was arranged between (D-2-2) and (D-3-2) to stack three layers in total, and embossing lamination integration was performed in accordance with the method as described in Example 2 in the condition that the heating temperatures for embossing and flat rollers were each 115°C, to give a mixed-fiber nonwoven fabric laminate.
The resulting mixed-fiber nonwoven fabric laminate was evaluated by the above-described methods. Table 2 shows the evaluation results.

Example 4

Production of mixed-fiber spunbonded nonwoven fabric (D-1-3)

Spunbonded nonwoven fabric (D-1-3) comprising only the continuous fibers A of the thermoplastic polyurethane elastomer (A-1) was produced in accordance with the method as described in Example 1.

Production of mixed-fiber spunbonded nonwoven fabrics (D-2-3) and (D-3-3)

Mixed-fiber spunbonded nonwoven fabrics (D-2-3) and (D-3-3) each comprising the continuous fibers A and the continuous fibers B at a fiber mixing ratio of 40:60 (weight ratio) and having a mass per unit area of 5 g/m$^2$ were produced in accordance with the method as described in Example 1.

Production of mixed-fiber nonwoven fabric laminate

Spunbonded nonwoven fabric layer (D-1-3) was arranged between (D-2-3) and (D-3-3) to laminate three layers in total, and embossing lamination integration was performed in accordance with the method as described in Example 2 in the condition that the temperatures for embossing and flat rollers were each 110°C, to give a mixed-fiber nonwoven fabric laminate.

The resulting mixed-fiber nonwoven fabric laminate was evaluated by the above-described methods. Table 2 shows the evaluation results.

Comparative Example 1

A single layer of mixed-fiber spunbonded nonwoven fabric comprising the continuous fibers A and the continuous fibers B at a fiber mixing ratio of 60:40 (weight ratio) and a mass per unit area of 30 g/m$^2$ was prepared and was embossed in the condition that the temperatures for both embossing and flat rollers were each 100°C, in accordance with the method as described in Example 1.

The resulting mixed-fiber nonwoven fabric was evaluated by the above-described methods. Table 2 shows the evaluation results.

[Table 1]

| Table 1 | |
|---|---|
| | Example 1 |
| Stickiness of C-2 surface before drawing | ○ |
| Stickiness of C-2 surface after drawing | ◎ |
| Residual strain MD | 12.5 |
| Residual strain CD | 22 |
| Maximum elongation MD | 160 |
| Maximum elongation CD | 150 |
| Fuzzing | none |

[Table 2]

Table 2

| | Exampl e 2 | Exampl e 3 | Exampl e 4 | Compara tive Example 1 |
|---|---|---|---|---|
| Stickiness of D-2 and D-3 surfaces before drawing | ○ | ◎ | ○ | Δ |
| Stickiness of D-2 and D-3 surfaces after drawing | ◎ | ◎ | ◎ | ○ |
| Residual strain MD | 11.8 | 13.5 | 10.5 | 12.2 |
| Residual strain CD | 21.5 | 24.9 | 18 | 22.3 |
| Maximum elongation MD | 163 | 159 | 150 | 161 |
| Maximum elongation CD | 159 | 166 | 155 | 162 |

(continued)

| | Exampl e 2 | Exampl e 3 | Exampl e 4 | Compara tive Example 1 |
|---|---|---|---|---|
| Fuzzing | none | none | none | none |
| Curling | none | none | none | none |

Industrial Applicability

The mixed-fiber nonwoven fabric laminate of the present invention is excellent in strechability, flexibility, moisture permeability, air permeability, fuzzing resistance, curling resistance, and strength and can be appropriately used in not only sanitary materials but also, for example, in medical materials, hygienic materials, and industrial materials by utilizing those characteristics. Specifically, examples of the sanitary materials include absorptive articles such as disposable diapers and sanitary napkins. In a spreading-type disposable diaper or a pants-type disposable diaper, the laminate can be appropriately used in portions such as a top sheet, a back sheet, a waistband (extension tape, side flap), a fastening tape, a three-dimensional gather, leg cuffs, and side panels of the pants-type disposable diaper. In a sanitary napkin, the laminate can be appropriately used in portions such as a top sheet, a back sheet, a wing, and cuffs for preventing side leakage. By using the laminate of the present invention in these portions, the absorptive articles can follow the movement of wearers and can fit the bodies of the wearers, and therefore comfortable conditions are maintained during the wearing, and also reductions in thickness, weight, and package size articles can be expected.
In the medical materials, the laminate can be used in various portions by utilizing the strechability suitable as poultice backing, satisfactory touch feeling, a property following movement of the body, and a skin-care property, and it can be further expected to provide a healing effect. Similarly, in the backing material for wound treatment, since the laminate has a suitable strechability to enhance adhesion to the affected area, it can be expected to provide an effect of enhancing wound recovery. Furthermore, the mixed-fiber nonwoven fabric laminate of the present invention has not only suitable air permeability similar to that of common nonwoven fabrics but also excellent strechability. Accordingly, the laminate can be expected to be used in portions that are required air permeability and strechability, such as disposable operating gown, cap, and movable portions, e.g., arms, elbows, a shoulder, and sleeves, of a rescue gown.

**Claims**

1. A mixed-fiber nonwoven fabric laminate comprising at least two mixed-fiber spunbonded nonwoven fabric layers whose fiber mixing ratios of continuous fibers of a thermoplastic elastomer (A) and continuous fibers of a thermoplastic resin (B) are different from each other.

2. The mixed-fiber nonwoven fabric laminate according to Claim 1, wherein the at least two mixed-fiber spunbonded nonwoven fabric layers comprises a mixed-fiber spunbonded nonwoven fabric layer (C-1) and a mixed-fiber spunbonded nonwoven fabric layer (C-2), the mixed-fiber spunbonded nonwoven fabric layer (C-1) comprising the continuous fibers of the thermoplastic elastomer (A) and the continuous fibers of the thermoplastic resin (B) at a fiber mixing ratio of 40 to 95 wt% : 60 to 5 wt% (wherein, (A)+(B)=100 wt%), the mixed-fiber spunbonded nonwoven fabric layer (C-2) comprising the continuous fibers of the thermoplastic elastomer (A) and the continuous fibers of the thermoplastic resin (B) at a fiber mixing ratio of 10 to 60 wt% : 90 to 40 wt% (wherein, (A)+(B)=100 wt%) .

3. A mixed-fiber nonwoven fabric laminate comprising at least three mixed-fiber spunbonded nonwoven fabric layers comprising continuous fibers of the thermoplastic elastomer (A) and continuous fibers of the thermoplastic resin (B), wherein the three mixed-fiber spunbonded nonwoven fabric layers are laminated such that the fiber mixing ratio of the thermoplastic elastomer (A) in the mixed-fiber spunbonded nonwoven fabric layer (intermediate layer) located at the middle of the three mixed-fiber spunbonded nonwoven fabric layers is larger than those of the thermoplastic elastomer (A) in the other two layers.

4. The mixed-fiber nonwoven fabric laminate according to Claim 3, wherein the intermediate layer is a mixed-fiber spunbonded nonwoven fabric layer (D-1), and the other two layers are a mixed-fiber spunbonded nonwoven fabric layer (D-2) and a mixed-fiber spunbonded nonwoven fabric layer (D-3), the mixed-fiber spunbonded nonwoven fabric layer (D-1) comprising the continuous fibers of the thermoplastic elastomer (A) and the continuous fibers of the thermoplastic resin (B) at a fiber mixing ratio of 40 to 100 wt% : 60 to 0 wt% (wherein, (A)+(B)=100 wt%), the mixed-fiber spunbonded nonwoven fabric layer (D-2) comprising the continuous fibers of the thermoplastic elastomer (A) and the continuous fibers of the thermoplastic resin (B) at a fiber mixing ratio of 10 to 60 wt% : 90 to 40 wt% (wherein, (A)+(B)=100 wt%), the mixed-fiber spunbonded nonwoven fabric layer (D-3) comprising the continuous fibers of the thermoplastic elastomer (A) and the continuous fibers of the thermoplastic resin (B) at a fiber mixing ratio of 10 to 60 wt% : 90 to 40 wt% (wherein, (A)+(B)=100 wt%).

5. The mixed-fiber nonwoven fabric laminate according to any of Claims 1 to 4, wherein the continuous fibers of the plastic elastomer (A) are continuous fibers of a thermoplastic polyurethane elastomer.

6. The mixed-fiber nonwoven fabric laminate according to any of Claims 1 to 4, wherein the continuous fibers of the thermoplastic elastomer (A) are continuous fibers of an olefin copolymer elastomer.

7. The mixed-fiber nonwoven fabric laminate according to Claims 6, wherein the continuous fibers of the olefin copolymer elastomer are continuous fibers of an ethylene/$\alpha$-olefin copolymer elastomer or continuous fibers of a propylene/$\alpha$-olefin copolymer elastomer.

8. The mixed-fiber nonwoven fabric laminate according to Claims 6, wherein the continuous fibers of the olefin copolymer elastomer are continuous fibers comprising a composition of an ethylene/$\alpha$-olefin copolymer elastomer and crystalline polyolefin, or continuous fibers comprising a composition of a propylene/$\alpha$-olefin copolymer elastomer and crystalline polyolefin.

9. The mixed-fiber nonwoven fabric laminate according to any of Claims 1 to 4, wherein the continuous fibers of the thermoplastic resin (B) in a spunbonded nonwoven fabric has a maximum elongation of 50% or more.

10. The mixed-fiber nonwoven fabric laminate according to any of Claims 1 to 4, wherein the continuous fibers of the thermoplastic resin (B) are continuous fibers of a propylene polymer.


**Patentansprüche**

1. Mischfaser-Vliesstofflaminat, das mindestens zwei Mischfaser-Spinnvliesschichten umfasst, deren Fasermischverhältnisse aus kontinuierlichen Fasern aus einem thermoplastischen Elastomer (A) und kontinuierlichen Fasern aus einem thermoplastischen Harz (B) voneinander verschieden sind.

2. Mischfaser-Vliesstofflaminat gemäß Anspruch 1, worin die mindestens zwei Mischfaser-Spinnvliesschichten eine Mischfaser-Spinnvliesschicht (C-1) und eine Mischfaser-Spinnvliesschicht (C-2) umfassen, wobei die Mischfaser-Spinnvliesschicht (C-1) kontinuierliche Fasern des thermoplastischen Elastomers (A) und kontinuierliche Fasern des thermoplastischen Harzes (B) bei einem Fasermischverhältnis von 40 bis 95 Gew.% : 60 bis 5 Gew.% umfasst (worin (A)+(B)=100 Gew.%) und die Mischfaser-Spinnvliesschicht (C-2) kontinuierliche Fasern des thermoplastischen Elastomers (A) und kontinuierliche Fasern des thermoplastischen Harzes (B) bei einem Fasermischverhältnis von 10 bis 60 Gew.% : 90 bis 40 Gew.% umfasst (worin (A)+(B)=100 Gew.%).

3. Mischfaser-Vliesstofflaminat, das mindestens drei Mischfaser-Spinnvliesschichten umfasst, die kontinuierliche Fasern des thermoplastischen Elastomers (A) und kontinuierliche Fasern des thermoplastischen Harzes (B) umfassen, worin die drei Mischfaser-Spinnvliesschichten so laminiert sind, dass das Fasermischverhältnis des thermoplastischen Elastomers (A) in der Mischfaser-Spinnvliesschicht (mittlere Schicht), die in der Mitte der drei Mischfaser-Spinnvliesschichten lokalisiert ist, größer als das des thermoplastischen Elastomers (A) in den anderen beiden Schichten ist.

4. Mischfaser-Vliesstofflaminat gemäß Anspruch 3, worin die mittlere Schicht eine Mischfaser-Spinnvliesschicht (D-1) ist, und die beiden anderen Schichten eine Mischfaser-Spinnvliesschicht (D-2) und eine Mischfaser-Spinnvliesschicht (D-3) sind, wobei die Mischfaser-Spinnvliesschicht (D-1) kontinuierliche Fasern des thermoplastischen Elastomers (A) und kontinuierliche Fasern des thermoplastischen Harzes (B) bei einem Fasermischverhältnis von 40 bis 100 Gew.% : 60 bis 0 Gew.% umfasst (worin (A)+(B)=100 Gew.%), die Mischfaser-Spinnvliesschicht (D-2) kontinuierliche Fasern des thermoplastischen Elastomers (A) und kontinuierliche Fasern des thermoplastischen Harzes (B) bei einem Fasermischverhältnis von 10 bis 60 Gew.% : 90 bis 40 Gew.% umfasst (worin (A)+(B)=100 Gew.%) und die Mischfaser-Spinnvliesschicht (D-3) kontinuierliche Fasern des thermoplastischen Elastomers (A) und kontinuierliche Fasern des thermoplastischen Harzes (B) bei einem Fasermischverhältnis von 10 bis 60 Gew.% : 90 bis 40 Gew.% umfasst (worin (A)+(B)=100 Gew.%).

**5.** Mischfaser-Vliesstofflaminat gemäß irgendeinem der Ansprüche 1 bis 4, worin die kontinuierlichen Fasern des thermoplastischen Elastomers (A) kontinuierliche Fasern eines thermoplastischen Polyurethan-Elastomers sind.

**6.** Mischfaser-Vliesstofflaminat gemäß irgendeinem der Ansprüche 1 bis 4, worin die kontinuierlichen Fasern des thermoplastischen Elastomers (A) kontinuierliche Fasern eines Olefin-Copolymer-Elastomers sind.

**7.** Mischfaser-Vliesstofflaminat gemäß Anspruch 6, worin die kontinuierlichen Fasern des Olefin-Copolymer-Elastomers kontinuierliche Fasern aus einem Ethylen/$\alpha$-Olefin-Copolymer-Elastomer oder kontinuierliche Fasern aus einem Propylen/$\alpha$-Olefin-Copolymer-Elastomer sind.

**8.** Mischfaser-Vliesstofflaminat gemäß Anspruch 6, worin die kontinuierlichen Fasern des Olefin-Copolymer-Elastomers kontinuierliche Fasern sind, die eine Zusammensetzung aus einem Ethylen/$\alpha$-Olefin-Copolymer-Elastomer und einem kristallinen Polyolefin umfassen, oder kontinuierliche Fasern, die eine Zusammensetzung aus einem Propylen/$\alpha$-Olefin-Copolymer-Elastomer und einem kristallinen Polyolefin umfassen, sind.

**9.** Mischfaser-Vliesstofflaminat gemäß irgendeinem der Ansprüche 1 bis 4, worin die kontinuierlichen Fasern des thermoplastischen Harzes (B) in einem Spinnvlies eine maximale Dehnung von 50 % oder mehr aufweisen.

**10.** Mischfaser-Vliesstofflaminat gemäß irgendeinem der Ansprüche 1 bis 4, worin die kontinuierlichen Fasern des thermoplastischen Harzes (B) kontinuierliche Fasern aus einem Propylenpolymer sind.

**Revendications**

**1.** Stratifié de tissu non-tissé à fibres mélangées comprenant au moins deux couches de tissu non-tissé filé-lié à fibres mélangées dont les rapports de mélange de fibres continues d'un élastomère thermoplastique (A) et de fibres continues d'une résine thermoplastique (B) sont différents les uns des autres.

**2.** Stratifié de tissu non-tissé à fibres mélangées selon la revendication 1, dans lequel les au moins deux couches de tissu non-tissé filé-lié à fibres mélangées comprennent une couche (C-1) de tissu non-tissé filé-lié à fibres mélangées et une couche (C-2) de tissu non-tissé filé-lié à fibres mélangées, la couche (C-1) de tissu non-tissé filé-lié à fibres mélangées comprenant les fibres continues de l'élastomère thermoplastique (A) et les fibres continues de la résine thermoplastique (B) à un rapport de mélange de fibres de 40 à 95% en poids : 60 à 5% en poids (où, (A) + (B) = 100% en poids), la couche (C-2) de tissu non-tissé filé-lié à fibres mélangées comprenant les fibres continues de l'élastomère thermoplastique (A) et les fibres continues de la résine thermoplastique (B) à un rapport de mélange de fibres de 10 à 60% en poids : 90 à 40% en poids (où, (A) + (B) = 100% en poids).

**3.** Stratifié de tissu non-tissé à fibres mélangées comprenant au moins trois couches de tissu non-tissé filé-lié à fibres mélangées comprenant des fibres continues de l'élastomère thermoplastique (A) et des fibres continues de la résine thermoplastique (B), où les trois couches de tissu non-tissé filé-lié à fibres mélangées sont stratifiées de sorte que le rapport de mélange de fibres de l'élastomère thermoplastique (A) dans la couche de tissu non-tissé filé-lié à fibres mélangées (couche intermédiaire) située au milieu des trois couches de tissu non-tissé filé-lié à fibres mélangées soit supérieur à celui de l'élastomère thermoplastique (A) dans les deux autres couches.

**4.** Stratifié de tissu non-tissé à fibres mélangées selon la revendication 3, dans lequel la couche intermédiaire est une couche (D-1) de tissu non-tissé filé-lié à fibres mélangées, et les deux autres couches constituent une couche (D-2) de tissu non-tissé filé-lié à fibres mélangées et une couche (D-3) de tissu non-tissé filé-lié à fibres mélangées, la couche (D-1) de tissu non-tissé filé-lié à fibres mélangées comprenant les fibres continues de l'élastomère thermoplastique (A) et les fibres continues de la résine thermoplastique (B) à un rapport de mélange de fibres de 40 à 100% en poids : 60 à 0% en poids (où, (A) + (B) = 100% en poids), la couche (D-2) de tissu non-tissé filé-lié à fibres mélangées comprenant les fibres continues de l'élastomère thermoplastique (A) et les fibres continues de la résine thermoplastique (B) à un rapport de mélange de fibres de 10 à 60% en poids : 90 à 40% en poids (où, (A) + (B) = 100% en poids), la couche (D-3) de tissu non-tissé filé-lié à fibres mélangées comprenant les fibres continues de l'élastomère thermoplastique (A) et les fibres continues de la résine thermoplastique (B) à un rapport de mélange de fibres de 10 à 60% en poids : 90 à 40% en poids (où, (A) + (B) = 100% en poids).

**5.** Stratifié de tissu non-tissé à fibres mélangées selon l'une des revendications 1 à 4, dans lequel les fibres continues de l'élastomère plastique (A) sont des fibres continues d'un élastomère de polyuréthanne thermoplastique.

6. Stratifié de tissu non-tissé à fibres mélangées selon l'une des revendications 1 à 4, dans lequel les fibres continues de l'élastomère thermoplastique (A) sont des fibres continues d'un élastomère de copolymère d'oléfine.

7. Stratifié de tissu non-tissé à fibres mélangées selon la revendication 6, dans lequel les fibres continues de l'élastomère de copolymère d'oléfine sont des fibres continues d'un élastomère de copolymère d'éthylène/$\alpha$-oléfine ou des fibres continues d'un élastomère de copolymère de propylène/$\alpha$-oléfine.

8. Stratifié de tissu non-tissé à fibres mélangées selon la revendication 6, dans lequel les fibres continues de l'élastomère de copolymère d'oléfine sont des fibres continues comprenant une composition d'un élastomère de copolymère d'éthylène/$\alpha$-oléfine et de polyoléfine cristalline, ou des fibres continues comprenant une composition d'un élastomère de copolymère de propylène/$\alpha$-oléfine et de polyoléfine cristalline.

9. Stratifié de tissu non-tissé à fibres mélangées selon l'une des revendications 1 à 4, dans lequel les fibres continues de la résine thermoplastique (B) dans un tissu non-tissé filé-lié présente un allongement maximal de 50% ou plus.

10. Stratifié de tissu non-tissé à fibres mélangées selon l'une des revendications 1 à 4, dans lequel les fibres continues de la résine thermoplastique (B) sont des fibres continues d'un polymère de propylène.

**EP 2 123 441 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 7503502 A
- JP 2004244791 A
- JP 2004197291 A